(19) Europäisches Patentamt
European Patent Office
Office européen des brevets

(11) **EP 2 554 115 B1**

(12) **EUROPEAN PATENT SPECIFICATION**

(45) Date of publication and mention
of the grant of the patent:
**25.02.2015 Bulletin 2015/09**

(51) Int Cl.:
*A61B 5/1455* *(2006.01)*    *A61B 5/00* *(2006.01)*

(21) Application number: **12176834.5**

(22) Date of filing: **18.07.2012**

(54) **Apparatus and method for acquiring information on subject**

Vorrichtung und Verfahren zur Erfassung von Informationen zu einer Person

Appareil et procédé pour l'acquisition d'informations sur un sujet

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.08.2011 JP 2011172061**

(43) Date of publication of application:
**06.02.2013 Bulletin 2013/06**

(73) Proprietor: **Canon Kabushiki Kaisha
Tokyo 146-8501 (JP)**

(72) Inventor: **Miyasato, Takuro
Tokyo, Tokyo 146-8501 (JP)**

(74) Representative: **TBK
Bavariaring 4-6
80336 München (DE)**

(56) References cited:
**US-A1- 2007 232 911    US-B1- 7 515 948**

• **MIN QU ET AL: "Combined photoacoustic and
magneto-motive ultrasound imaging",
PROCEEDINGS OF THE SPIE - THE
INTERNATIONAL SOCIETY FOR OPTICAL
ENGINEERING SPIE - THE INTERNATIONAL
SOCIETY FOR OPTICAL ENGINEERING USA, vol.
7564, 2010, XP040548853, ISSN: 0277-786X**

**Description**

BACKGROUND OF THE INVENTION

Field of the Invention

The present invention relates to an apparatus and method for acquiring information such as a spectral characteristic of a subject by detecting an acoustic wave generated in a subject.

Description of the Related Art

Photoacoustic tomography is attracting attention as a technique to obtain an image uniquely representing a new blood vessel formed by cancer. The PAT is a technique to illuminate a subject with a pulsed light (a near infrared ray), detect a photoacoustic wave generated by the subject with an acoustic wave detector, and convert the detected photoacoustic wave into an image.

The initial pressure $P_0$ of the photoacoustic wave generated in a region of interest of the subject can be expressed by Equation (1) shown below.

$$P_0 = \Gamma \cdot \mu_a \cdot \Phi \qquad (1)$$

In this Equation, $\Gamma$ is a Gruneisen coefficient which is obtained by dividing the product of the coefficient of volume expansion $\beta$ and the square of the sound speed c by the specific heat $C_p$. It is known that when a subject is given, $\Gamma$ takes a substantially constant value for the given object. $\mu_a$ is an absorption coefficient of a region of interest, $\Phi$ is the light intensity in the region of interest (the light intensity of light illuminating the region of interest, which is also called light fluence).

The photoacoustic wave generated in the subject propagates through the subject and is detected by an acoustic wave detector put on the surface of the subject. Based on a detection result, a signal processing apparatus acquires an initial pressure distribution $P_0$ by using a reconstruction method such as a back projection method or the like.

From Equation (1), by dividing the initial pressure distribution $P_0$ by the Gruneisen coefficient $\Gamma$, the distribution of the product of $\mu_a$ and $\Phi$, i.e., an optical energy density distribution is obtained. Furthermore, an absorption coefficient distribution $\mu_a(r)$ can be obtained by dividing the optical energy density distribution by the light intensity distribution $\Phi(r)$ in the subject.

In a paper, "Functional Photoacoustic tomography for non-invasive imaging of cerebral blood oxygenation and blood volume in rat brain in vivo" (X. Wang, L. V. Wang et al. Proc. of SPIE Vol. 5697 (2005) (hereinafter referred to as NPL 1), a technique is disclosed to calculate an oxygen saturation distribution using two absorption coefficient distributions obtained for light with two wavelengths.

Let $\varepsilon_{HbO}$ ($mm^{-1}M^{-1}$) denote the molar absorption coefficient of oxyhemoglobin, and $\varepsilon_{Hb}$ ($mm^{-1}M^{-1}$) denote the molar absorption coefficient of deoxyhemoglobin. Note that the molar absorption coefficient refers to an absorption coefficient for a sample including 1 mol hemoglobin per 1 liter. The value of the molar absorption coefficient is uniquely determined by the wavelength.

Furthermore, let $C_{HbO}$ denote the molar concentration (M) of oxyhemoglobin, and $C_{Hb}$ denote the molar concentration (M) of deoxyhemoglobin. When parameters are given as described above, the absorption coefficient $\mu_a$ of blood at the wavelength $\lambda_1$ and that at the wavelength $\lambda_2$ are given by Equation (2) shown below.

$$\begin{cases} \mu_a(\lambda_1) = \varepsilon_{HbO}(\lambda_1) \cdot C_{HbO} + \varepsilon_{Hb}(\lambda_1) \cdot C_{Hb} \\ \mu_a(\lambda_2) = \varepsilon_{HbO}(\lambda_2) \cdot C_{HbO} + \varepsilon_{Hb}(\lambda_2) \cdot C_{Hb} \end{cases} \qquad (2)$$

As can be seen, the absorption coefficient $\mu_a$ of blood at each wavelength is given by the sum of the product of the molar absorption coefficient $\varepsilon_{HbO}$ of oxyhemoglobin and the molar concentration $C_{HbO}$ of oxyhemoglobin and the product of the molar absorption coefficient $\varepsilon_{Hb}$ of deoxyhemoglobin and the molar concentration $C_{Hb}$ of deoxyhemoglobin.

Equation (2) can be rewritten as described below in Equation (3).

$$\begin{cases} C_{HbO} = \dfrac{\varepsilon_{Hb}(\lambda_1)\cdot\mu_a(\lambda_2)-\varepsilon_{Hb}(\lambda_2)\cdot\mu_a(\lambda_1)}{\varepsilon_{Hb}(\lambda_1)\cdot\varepsilon_{HbO}(\lambda_2)-\varepsilon_{HbO}(\lambda_1)\cdot\varepsilon_{Hb}(\lambda_2)} \\[2mm] C_{Hb} = \dfrac{\varepsilon_{Hb}(\lambda_1)\cdot\mu_a(\lambda_2)-\varepsilon_{HbO}(\lambda_2)\cdot\mu_a(\lambda_1)}{\varepsilon_{HbO}(\lambda_1)\cdot\varepsilon_{Hb}(\lambda_2)-\varepsilon_{Hb}(\lambda_1)\cdot\varepsilon_{Hb}(\lambda_2)} \end{cases} \quad (3)$$

The oxygen saturation $StO_2$ is the ratio of oxyhemoglobin to the total hemoglobin, and thus it can be determined from Equation (3) as expressed in Equation (4) shown below.

$$StO_2 = \frac{C_{HbO}}{C_{HbO}+C_{Hb}} = \frac{-\mu_a(\lambda_1)\varepsilon_{Hb}(\lambda_2)+\mu_a(\lambda_2)\varepsilon_{Hb}(\lambda_1)}{-\mu_a(\lambda_1)\{\varepsilon_{Hb}(\lambda_2)-\varepsilon_{HbO}(\lambda_2)\}+\mu_a(\lambda_2)\{\varepsilon_{Hb}(\lambda_1)-\varepsilon_{HbO}(\lambda_1)\}}$$

$$(4)$$

That is, if the absorption coefficient $\mu_a$ of the blood at the wavelength $\lambda_1$ and that at the wavelength $\lambda_2$ are given, then all values are known and thus the oxygen saturation can be calculated from Equation (4).

In NPL 1, it is assumed that the light intensity constantly attenuates in a direction from a surface illuminated pulsed light to a target. Based on this assumption, the light intensity at the target is calculated, and the absorption coefficient of the target is determined using the calculated light intensity.

Japanese Patent Laid-Open No. 2010-88627 (hereinafter referred to as PTL 1) discloses a technique of acquiring an absorption coefficient using a light intensity distribution acquired taking into account the shape of a subject. The light intensity distribution obtained in this manner is more accurate than that according to NPL 1.

Prior art which is related to this field of technology can be found in e.g. document US 2007/0232911 A1 disclosing a device for photodetecting tumor, in non-patent literature Min Qu et. al., Proceedings of the SPIE - The International Society for Optical Engineering SPIE, vol. 7564, 2010, disclosing a combined photoacoustic and magneto-motive ultrasound imaging, and in document US 7 515 948 B1 disclosing a photoacoustic analyzer of region of interest in a human body.

SUMMARY OF THE INVENTION

By applying the technique of acquiring the absorption coefficient disclosed in PTL 1 to the technique of acquiring the oxygen saturation disclosed in NPL 1, it becomes possible to acquire the oxygen saturation with better accuracy.

However, although the technique disclosed in PTL 1 allows it to acquire the light intensity distribution with better accuracy, it needs an additional process for acquiring the light intensity distribution.

In view of the above, an embodiment provides a subject information acquisition apparatus capable of acquiring subject information with a less number of steps of acquiring a light intensity distribution.

The present invention provides a subject information acquisition apparatus as specified in claim 1.

The present invention also provides a method of acquiring subject information as specified in claim 10.

The present invention also provides a program as specified in claim 18. Preferred embodiments are defined in the dependent claims.

Further features of the present invention will become apparent from the following description of exemplary embodiments with reference to the attached drawings.

BRIEF DESCRIPTION OF THE DRAWINGS

Fig. 1 is a diagram illustrating a subject information acquisition apparatus according to a first embodiment.
Fig. 2 is a flow chart illustrating a subject information acquisition method according to the first embodiment.
Fig. 3 is a diagram illustrating an effective attenuation coefficient spectrum of a subject according to the first embodiment.

Fig. 4 is a diagram illustrating a monitor display screen according to the first embodiment.

Fig. 5 is a diagram illustrating absorption coefficient spectra of components of a subject according to the first embodiment.

Fig. 6 is a diagram illustrating a reduced scattering coefficient spectrum of a subject according to the first embodiment.

Fig. 7 is a diagram illustrating effective attenuation coefficient spectra of various objects with different component ratios under examination.

Fig. 8 is a diagram illustrating a subject information acquisition apparatus according to a second embodiment.

Fig. 9 is a flow chart illustrating a subject information acquisition method according to the second embodiment.

Fig. 10 is a flow chart illustrating a subject information acquisition method according to a third embodiment.

DESCRIPTION OF THE EMBODIMENTS

First Embodiment

An embodiment of a subject information acquisition apparatus is described below in detail with reference to drawings.

Basic Configuration

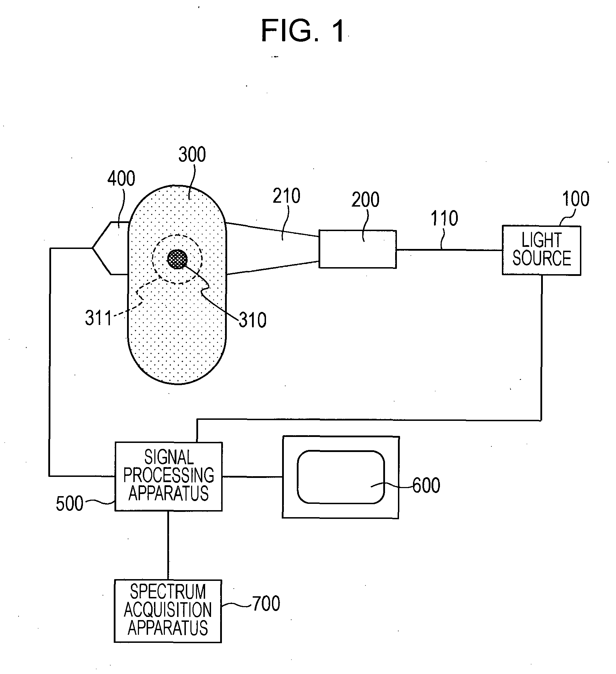

Fig. 1 illustrates a subject information acquisition apparatus according to the present embodiment.

The subject information acquisition apparatus according to the present embodiment includes, as basic hardware components, a light source 100, an illumination optical system 200 serving as an illumination optical unit, an acoustic wave detector 400 serving as an acoustic wave detection unit, a signal processing apparatus 500 serving as a signal processing unit, and a spectrum acquisition apparatus 700 serving as a spectrum acquisition unit. The light source 100 is configured to generate light with a first wavelength $\lambda_1$ and light with a second wavelength $\lambda_2$.

Pulsed light 110 with the first wavelength emitted from the light source 100 is passed through the illumination optical system 200 and resultant illumination light 210 illuminates a subject 300. A light absorber 310 in the subject 300 absorbs the illumination light 210 and generates a photoacoustic wave 311. The acoustic wave detector 400 detects the photoacoustic wave and converts it into a first detection signal in the form of an electric signal.

Similarly, the acoustic wave detector 400 detects a photoacoustic wave 311 generated by illuminating the subject 300 with illumination light 210 with the second wavelength and the acoustic wave detector 400 converts it into a second detection signal in the form of an electric signal.

Next, the signal processing apparatus 500 acquires an oxygen saturation distribution in the subject 300 based on the first detection signal and the second detection signal. The resultant oxygen saturation is displayed on a monitor 600.

Furthermore, in the present embodiment, the subject information acquisition apparatus also includes a spectrum acquisition apparatus 700 configured to acquire an effective attenuation coefficient spectrum of the subject 300. Based on the effective attenuation coefficient spectrum of the subject 300 acquired by the spectrum acquisition apparatus 700, the signal processing apparatus 500 selects the first wavelength and the second wavelength such that the same effective attenuation coefficient is obtained at these two wavelengths.

As described above, by acquiring the oxygen saturation using light with the first wavelength and light with the second wavelength at which the same effective attenuation coefficient is obtained, it becomes possible to reduce the number of steps of acquiring the light intensity.

Note that in the present embodiment, the average attenuation coefficient in the subject 300 is used as the effective attenuation coefficient. Furthermore, in the present embodiment, the subject information may include other information in addition to the oxygen saturation determined based on absorption coefficients at a plurality of wavelengths.

Next, a subject information acquisition method according to the present embodiment is described below.

Subject information acquisition method

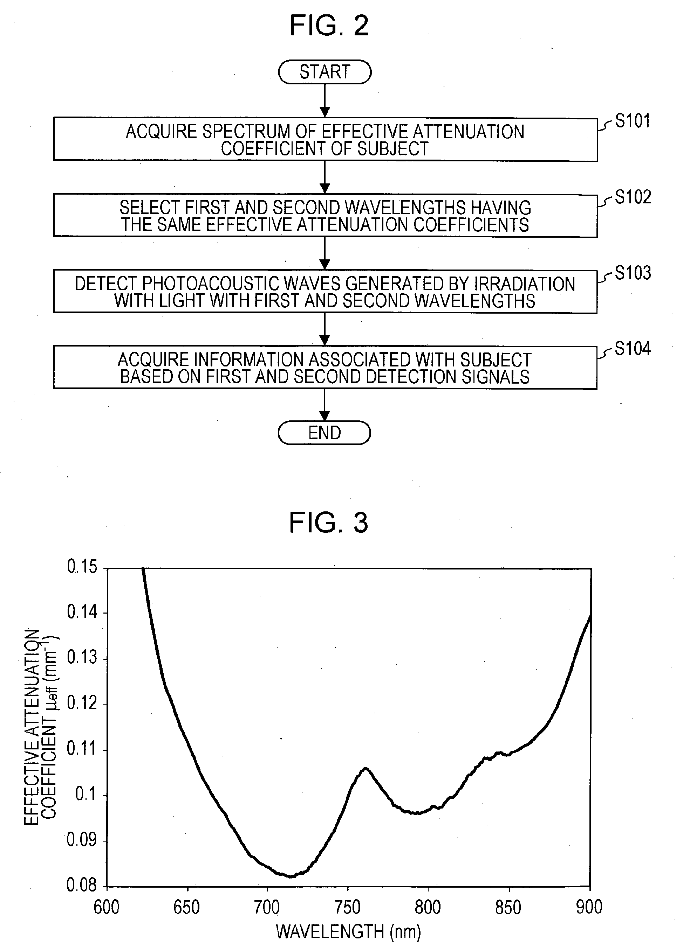

Referring to Fig. 1 and Fig. 2, the subject information acquisition method according to the present embodiment is described below. Fig. 2 is a flow chart illustrating the subject information acquisition method according to the present embodiment. Note that step numbers used in the following description correspond to step numbers shown in Fig. 2.

S101: Step of acquiring effective attenuation coefficient spectrum of subject

In this step, the spectrum acquisition apparatus 700 acquires the effective attenuation coefficient spectrum of the subject 300 shown in Fig. 3. The effective attenuation coefficient spectrum may be represented by a graph in which the wavelength is plotted horizontally and the effective attenuation coefficient $\mu_{eff}$ is plotted vertically. Note that the term "effective attenuation coefficient spectrum" may also be used to describe a set of data representing the graph described above. In Fig. 3, an effective attenuation coefficient spectrum is plotted for a particular subject 300 having a component ratio fat = 66% : water = 20% : blood = 0.7% (oxygen saturation = 80%).

S102: Step of selecting first wavelength and second wavelength at which the same effective attenuation coefficient is obtained

In this step, based on the effective attenuation coefficient spectrum acquired in step S101, the signal processing apparatus 500 selects two wavelengths at which the same effective attenuation coefficient is obtained. Note that in the present embodiment, the two wavelengths at which the same effective attenuation coefficient is obtained may be arbitrary two wavelengths as long as effective attenuation coefficients at these two wavelengths satisfy the requirement of the present embodiment, i.e., it is sufficient if the effective attenuation coefficients are substantially equal to each other.

In a case where the subject 300 is a living body, when it is desired to observe deep points thereof, the signal processing apparatus 500 may select wavelengths at which low effective attenuation coefficients are obtained. For example, wavelengths may be selected in a range from 650 nm to 900 nm, which is called a "living body window" because light with a wavelength in this range can easily pass through a living body.

Furthermore, taking into account a measurement error or measurement noise, the two wavelengths $\lambda_1$ and $\lambda_2$ may be selected such that the ratio of the absorption coefficient at a location of a blood vessel or cancer between the two wavelengths $\lambda_1$ and $\lambda_2$, i.e., $\mu_a(\lambda_1)/\mu_a(\lambda_2)$, changes as much as possible with a change in the oxygen saturation. More specifically, for example, when the molar absorption coefficient of deoxyhemoglobin at the wavelength $\lambda_1$ is given by $\varepsilon_{Hb}(\lambda_1)$ and that at the wavelength $\lambda_2$ is given by $\varepsilon_{Hb}(\lambda_2)$, and the molar absorption coefficient of oxyhemoglobin at the wavelength $\lambda_1$ is given by $\varepsilon_{HbO}(\lambda_1)$ and that at the wavelength $\lambda_2$ is given by $\varepsilon_{HbO}(\lambda_2)$, then the signal processing apparatus 500 may select the wavelengths $\lambda_1$ and $\lambda_2$ such that $|(\varepsilon_{HbO}(\lambda_1) - \varepsilon_{Hb}(\lambda_1) - (\varepsilon_{HbO}(\lambda_2) - \varepsilon_{Hb}(\lambda_2))|$ is maximized.

As for the laser used in the PAT, a Ti:Sa laser, an alexandrite laser, or the like may be used. The Ti:Sa laser provides a maximum output power in a range around 800 nm, while the alexandrite laser provides a maximum output power in a range around 755 nm. The signal processing apparatus 500 may select wavelengths in a range from 700 to 850 nm including the above-described ranges in which the respective lasers have their maximum output power. For example, the signal processing apparatus 500 may select the first wavelength in a range of 756 to 761 nm corresponding to a peak at 756 nm of deoxyhemoglobin and a peak at 761 nm of fat. The signal processing apparatus 500 may select the second wavelength in a range of 830 to 840 nm corresponding to a peak at 830 nm of fat and a peak at 840 nm of water.

The two wavelengths at which the effective attenuation coefficient is equal may be defined as follows.

The oxygen saturation of an artery is typically 99%, and the oxygen saturation of a vein is typically 80%. According to a paper titled "Combined diffuse optical spectroscopy and contrast-enhanced magnetic resonance imaging for monitoring breast cancer neoadjuvant chemotherapy: a case study" (Natasha Shah, Jessica Gibbs, Dulcy Wolverton, Albert Cerussi, Nola Hylton, Bruce J Tromberg, Journal of Biomedical Optics (2005) Volume: 10, Issue: 5, Pages: 051503), the oxygen saturation of a new blood vessel generated by a tumor can be estimated as 66%. Therefore, if the error of the oxygen saturation is within a range of $\pm 10\%$, it is possible to distinguish between a new blood vessel and a normal blood vessel. That is, the two wavelengths at which the effective attenuation coefficient is equal may be selected from a range in which the error of the oxygen saturation calculated for a particular measurement depth is within $\pm 10\%$. To calculate the oxygen saturation with better accuracy, wavelengths may be selected such that the error of the oxygen saturation is less than $\pm 10\%$.

In the following discussion, let it be assumed, by way of example, that the subject has a plurality of light absorbers located at depths 0 to 30 mm. Let it be further assumed that each light absorber has an oxygen saturation in a range from 50 to 100%, the difference in oxygen saturation between any two light absorbers is 10% at most, and the subject has an effective attenuation coefficient of 0.09233 mm$^{-1}$ in a wavelength range of 756 nm to 797 nm.

In such a situation, to distinguish among the absorbers, Equation (4) indicates that a necessary difference in the effective attenuation coefficient between the two wavelengths is $\pm 0.003$ mm$^{-1}$. The light intensity $\Phi(d)$ in the subject is determined according to a one-dimensional model shown below in Equation (5), where $\Phi_0$ is the light intensity incident on the subject, $\mu_{eff}$ is the effective attenuation coefficient of the subject, and d is the measurement depth.

$$\Phi(d) = \Phi_0 \cdot \exp\left(- \mu_{eff} \cdot d\right) \qquad (5)$$

From the above discussion, it can be seen that when 756 nm is selected as the first wavelength, a necessary effective attenuation coefficient for the second wavelength is 0.09233 $\pm$ 0.003 mm$^{-1}$. That is, when 756 nm is selected as the first wavelength, the second wavelength is not limited to exactly 797 nm as long as it is possible to distinguish between absorbers with oxygen saturations that are different by 10%.

That is, in the present embodiment, the two wavelengths with the same effective attenuation coefficient do not necessarily need to be exactly equal in the effective attenuation coefficient, but two wavelengths may be regarded as having equal effective attenuation coefficients if the two wavelengths allow it to distinguish between a normal blood vessel and a new blood vessel produced by a tumor. For example, as in the present embodiment, two wavelengths may be regarded as being equal in effective attenuation coefficient if these two wavelengths allow it to distinguish between absorbers having oxygen saturations different by 10%.

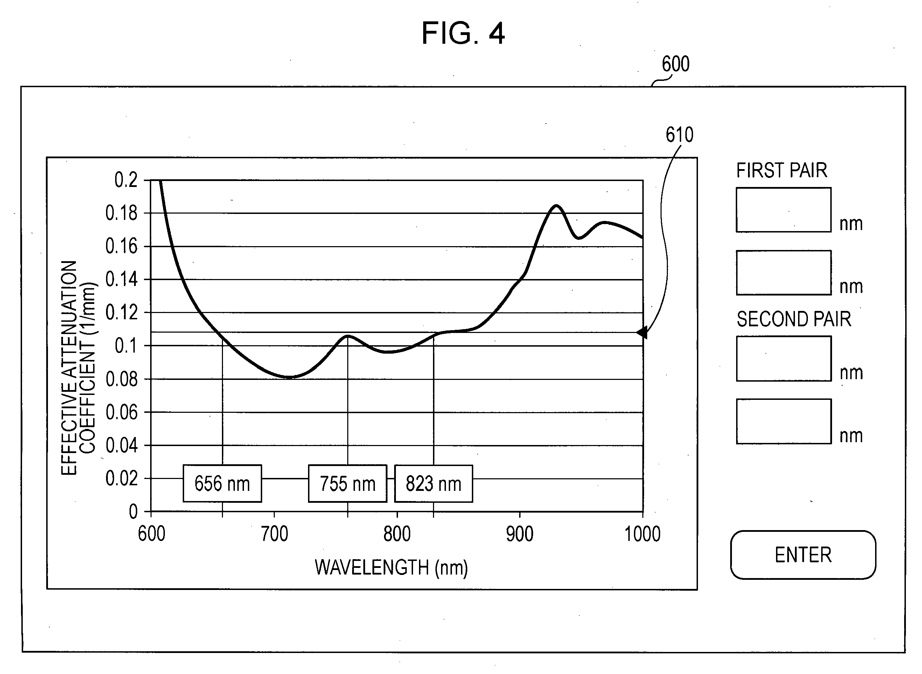

As shown in Fig. 4, the effective attenuation coefficient spectrum acquired in step S101 may be displayed on the monitor 600 such that a user is allowed to select two wavelengths, which give the same effective attenuation coefficient, from the displayed effective attenuation coefficient spectrum.

In the above-described example, two wavelengths are selected such that equal effective attenuation coefficients are obtained at the two wavelengths. Alternatively, as shown in Fig. 4, the signal processing apparatus 500 may select three wavelengths at which the effective attenuation coefficient is equal to a particular value denoted by reference numeral 610. In the example shown in Fig. 4, three wavelengths 656 nm, 755 nm, and 823 nm are selected. Note that in the present embodiment, four or more wavelengths may be selected at which effective attenuation coefficients are equal, if such a selection is possible.

Still alternatively, the signal processing apparatus 500 may select a first pair of wavelengths with a particular effective attenuation coefficient value 610 and may further select a second pair of wavelengths with another effective attenuation coefficient value different from the effective attenuation coefficient value 610.

Note that any method may be employed to select wavelengths from an effective attenuation coefficient spectrum as long as it is possible to select wavelengths at which effective attenuation coefficients are equal.

S103: Step of detecting photoacoustic wave generated by illumination of light with first and second wavelengths

In this step, the light source 100 generates pulsed light 110 with the two wavelengths selected in step S102. The pulsed light 110 is guided by the illumination optical system 200 such that the illumination light 210 strikes the subject 300. The acoustic wave detector 400 detects a photoacoustic wave 311 generated by the illumination of light with the respective wavelengths.

More specifically, in the present embodiment, the acoustic wave detector 400 detects a photoacoustic wave generated by illuminating the subject 300 with the light with the first wavelength and output an electric signal as a first detection signal, and the acoustic wave detector 400 detects a photoacoustic wave generated by illuminating the subject 300 with the light with the second wavelength and output an electric signal as a second detection signal.

S104: Step of acquiring subject information based on first and second detection signals

In this step, based on the first detection signal and the second detection signal acquired in step S103, the signal processing apparatus 500 acquires, as subject information, an oxygen saturation distribution in the subject 300.

More specifically, first, the signal processing apparatus 500 performs an image reconstruction based on the first detection signal and acquires a first initial pressure distribution $P_0(\lambda_1, r)$ in the subject 300. Similarly, the signal processing apparatus 500 performs an image reconstruction based on the second detection signal and acquires a second initial pressure distribution $P_0(\lambda_2, r)$ in the subject 300.

From Equation (1), the initial pressure distribution obtained in the above-described manner can be expressed as in Equation (6) shown below.

$$\begin{cases} P_0(\lambda_1, r) = \Gamma \cdot \mu_a(\lambda_1, r) \cdot \Phi(\lambda_1, r) \\ P_0(\lambda_2, r) = \Gamma \cdot \mu_a(\lambda_2, r) \cdot \Phi(\lambda_2, r) \end{cases} \quad (6)$$

That is, the initial pressure distribution $P_0$ is given by the product of the Gruneisen coefficient $\Gamma$, the absorption coefficient distribution $\mu_a$ in the subject 300, and the light intensity distribution $\Phi$ in the subject 300.

In the present embodiment, the two wavelengths are selected such that the same effective attenuation coefficient of the subject 300 is obtained at these two wavelengths, and thus the same light intensity distribution in the subject 300 is obtained for both wavelengths.

Since the same light intensity distribution is obtained for both wavelengths, by applying the initial pressure in Equation (6) to Equation (4), Equation (7) is obtained which represents the oxygen saturation.

$$StO_2(r) = \frac{-P_0(\lambda_1, r) \cdot \varepsilon_{Hb}(\lambda_2) + P_0(\lambda_2, r)\varepsilon_{Hb}(\lambda_1)}{-P_0(\lambda_1, r)\{\varepsilon_{Hb}(\lambda_2) - \varepsilon_{HbO}(\lambda_2)\} + P_0(\lambda_2, r)\{\varepsilon_{Hb}(\lambda_1) - \varepsilon_{HbO}(\lambda_1)\}}$$

$$(7)$$

From Equation (7), the signal processing apparatus 500 is capable of calculating the oxygen saturation only from the initial pressure without using the light intensity distribution. That is, in the subject information acquisition method according to the present embodiment, it is allowed to remove the process of acquiring the light intensity in the process of acquiring the oxygen saturation.

Note that the signal processing apparatus 500 may first determine the initial pressure at respective points in the subject 300 based on the first detection signal and the second detection signal, and then may acquire the oxygen saturation at the respective points from the initial pressure at the points, and may finally acquire the oxygen saturation distribution.

In the present embodiment, the signal processing apparatus 500 may acquire the oxygen saturation based on the detection signal using a method other than the method of calculating the oxygen saturation other than Equation (7). For example, based on the detection signal, the signal processing apparatus 500 may retrieve an oxygen saturation from an oxygen saturation data table stored in advance in a memory of the signal processing apparatus 500 whereby the signal processing apparatus 500 may acquire the oxygen saturation.

The signal processing apparatus 500 may be implemented on a computer, and a program including the process described above may be executed by the computer.

It is possible to acquire the oxygen saturation by performing the process described above without using the light intensity, and thus the process of acquiring the light intensity is no longer necessary unlike in other known techniques. Therefore, in the subject information acquisition apparatus according to the present embodiment, it is possible to remove measurement equipment for acquiring the light intensity, and thus it is possible to reduce the total apparatus size. Furthermore, it is possible to reduce the error in the measurement for determining the absorption coefficient, and thus it is possible to obtain more accurate oxygen saturation.

Furthermore, in the present embodiment, it is possible to acquire accurate oxygen saturation even in a case where it is difficult to measure the illumination light distribution or the shape of the subject or even in a case where it is difficult to define the shape by the subject by a holding plate or the like.

A specific example of a configuration of the present embodiment is described below. Light source

The light source 100 may be configured to generate pulsed light with a pulse width of 5 nsec to 50 nsec. To make it possible to determine the oxygen saturation distribution at a depth of a several ten mm, the output power of the light source 100 may be a few mJ/pulse or greater. A high-power laser or light emitting diode may be used as the light source 100. As for the laser, many types of lasers such as a solid-state laser, a gas laser, a dye laser, a semiconductor laser, etc. may be employed. More specifically, for example, a Ti:Sa laser pumped by Nd:YAG, an alexandrite laser, or the like capable of outputting high power and capable of continuously changing the wavelength may be used.

The light source 100 may be realized by a combination of a plurality of single-wavelength lasers with different wavelengths. For example, the light source 100 may include a first light source configured to generate light with the first wavelength and a second light source configured to generate light with the second wavelength.

Illumination optical system

The illumination optical system 200 is an optical system that guides pulsed light generated by the light source 100 such that the pulsed light strikes, as illumination light 210, the subject 300. For example, the illumination optical system 200 may include a light reflection mirror, a half mirror that splits the light into reference light and illumination light, a lens that focuses the light, enlarges the light, or change the shape of the light, a diffusion plate that expands light, etc. The illumination light 210 may be expanded by a lens so as to have a proper cross-sectional area. The illumination light 210 may be converted by a diffusion plane, a fly-eye lens, or the like into light with a smooth light intensity distribution.

The illumination optical system 200 may be configured to illuminate the subject 300 with the illumination light 210 such that the illumination light 210 provides the same optical pattern for all wavelengths used. In a case where a bundle fiber is used as the illumination optical system 200, output ends of element fibers of the bundle fiber may be arranged at random with respect to the incident ends such that an equal optical pattern is obtained for any wavelength.

The illumination area of the illumination light 210 may move on the subject 300. By moving the illumination area such that light illuminates the subject 300 in an overlapping manner, it is possible to obtain a uniform light intensity distribution over the surface of the subject. This situation is equivalent to the situation in which the subject is illuminated with two light rays having different wavelengths and having equal light intensity distributions. The illumination area on the subject 300 may be moved by using a movable mirror or by mechanically moving the light source itself.

The illumination light 210 may strike the subject 300 from the side of the acoustic wave detector 400 or from the opposite side. The illumination light 210 may strike both sides of the subject 300.

Acoustic wave detector

The acoustic wave detector 400 is configured to detect a photoacoustic wave 311 generated by the light absorber 310. As for the acoustic wave detector, a transduce using a piezoelectric effect, a transducer using resonance of light, a transduce using a change in capacitance, or the like may be used. Note that any other type of transduce may be used as the acoustic wave detector as long as it is capable of detecting photoacoustic waves. The transducer may be of an array type or a single-element type. The acoustic wave detector 400 may be installed at a fixed location to detect the photoacoustic wave or may be installed such that the acoustic wave detector 400 is scanned along the subject 300. Alternatively, the acoustic wave detector 400 may be moved around the subject 300 to detect photoacoustic waves at various points.

Signal processing apparatus

The signal processing apparatus 500 is an apparatus configured to acquire subject information based on the detection signal supplied from the acoustic wave detector 400.

The signal processing apparatus 500 may amplify the detection signal supplied from the acoustic wave detector 400 and may convert the amplified detection signal from an analog signal into a digital signal.

Note that in the present description, the term "detection signal" is used to describe both the analog signal output from the acoustic wave detector 400 and the digital signal converted from the analog signal.

The signal processing apparatus 500 further acquires an optical characteristic value distribution in the subject by reconstructing an image based on the detection signal. The signal processing apparatus 500 is typically a workstation or the like configured to execute software including a program prepared in advance in terms of the image reconstruction process and the like.

As for an image reconstruction algorithm, for example, inverse projection in time domain or Fourier domain may be used, which is widely used in tomography techniques.

In a case where it is allowed to spend much time on the reconstruction, the signal processing apparatus 500 may employ an image reconstruction method including iterative processing based on inverse problem analysis or the like.

By employing an acoustic wave detector including an acoustic lens or the like, it becomes possible for the signal processing apparatus 500 to acquire an initial pressure distribution in the subject without performing the image reconstruction.

In a case where a plurality of detection signals are obtained from the acoustic wave detector 400, the signal processing apparatus 500 may be configured to simultaneously process the plurality of signals such that forming an image is complete for a shorter time.

The signal processing apparatus 500 may be realized by a combination of separate apparatuses such as an amplifier, an analog-to-digital converter, an FPGA (Field Programmable Gate Array) chip, etc.

The signal processing described above may be implemented in a program, and the signal processing apparatus 500 may execute the program to perform the signal processing.

Spectrum acquisition apparatus

The spectrum acquisition apparatus 700 is an apparatus configured to acquire an effective attenuation coefficient spectrum associated with the subject 300.

As for the spectrum acquisition apparatus 700, for example, a diffuse optical spectroscopy (DOS) apparatus may be used.

DOS is a technique of illuminating a subject with light, detecting light propagating and diffusing in the subject with a photodetector, and obtaining optical constants (absorption coefficient $\mu_a$ and reduced scattering coefficient $\mu_s'$) of the subject from a light detection signal.

First, the DOS apparatus solve an inverse problem of the light propagation on the light detection signal detected by the photodetector, and calculates the average absorption coefficient $\mu_a$ and reduced scattering coefficient $\mu_s'$ of the subject. Next, the DOS apparatus evaluates the similarity, by means of fitting, of the calculated absorption coefficient and reduced scattering coefficient with respect to known absorption coefficient spectra of fat, water, oxyhemoglobin, deoxyhemoglobin, etc. The DOS apparatus determines a component ratio of the subject based on the result of fitting. Using the obtained component ratio of the subject, the DOS apparatus acquires an effective attenuation coefficient spectrum of the subject according to Equation (8).

$$\mu_{eff} = \sqrt{3\mu_a \cdot (\mu_s' + \mu_a)} \qquad (8)$$

In a case where the absorption coefficient is sufficiently smaller compared with the reduced scattering coefficient, the effective attenuation coefficient can be expressed by Equation (9).

$$\mu_{eff} = \sqrt{3\mu_a \cdot \mu_s'} \qquad (9)$$

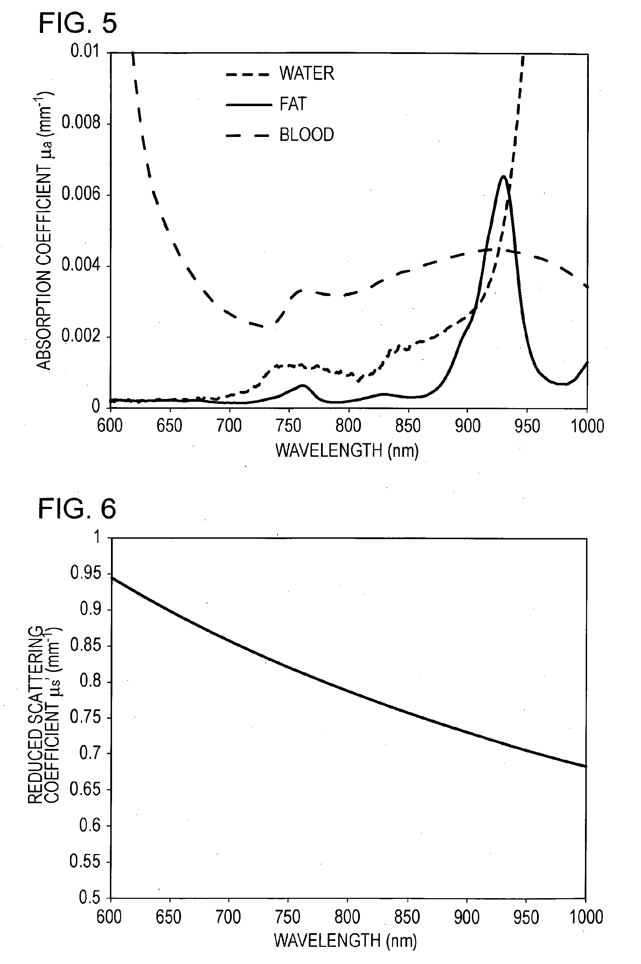

Fig. 5 shows absorption coefficient spectra of respective components obtained by fitting for a subject including fat (66%), water (20%), and blood (0.7%) of blood (oxygen saturation = 80%). Fig. 6 shows a typical reduced scattering coefficient spectrum of a human breast.

Thereafter, based on the absorption coefficient spectra shown in Fig. 5 and the reduced scattering coefficient spectrum shown in Fig. 6, the DOS apparatus acquires an effective attenuation coefficient spectrum from Equation (8) such as that shown in Fig. 3.

As for a technique of the DOS measurement, time-resolved diffuse optical spectroscopy (TR-DOS) using pulsed light, frequency-resolved diffuse optical spectroscopy (FR-DOS) using modulated light, or the like may be used. In the present embodiment, any DOS measurement technique may be used to acquire the effective attenuation coefficient spectrum.

Alternatively, optical constant distributions (an absorption coefficient distribution and a scattering coefficient distribution) in the subject may be acquired using diffuse optical tomography (DOT) or the like, and an average attenuation coefficient in an arbitrary region of the subject may be employed as the effective attenuation coefficient.

The spectrum acquisition apparatus 700 may be disposed in the subject information acquisition apparatus according to the present embodiment or may be disposed separately from the subject information acquisition apparatus. For example, based on a light detection signal obtained using a photodetector provided separated from the subject information acquisition apparatus, the signal processing apparatus 500 may acquire the effective attenuation coefficient spectrum of the subject 300.

Alternatively, the spectrum acquisition apparatus 700 may acquire the effective attenuation coefficient spectrum by estimating the component ratio of the subject based on information on subject such as an age, a height, a weight, a body fat percentage, etc.

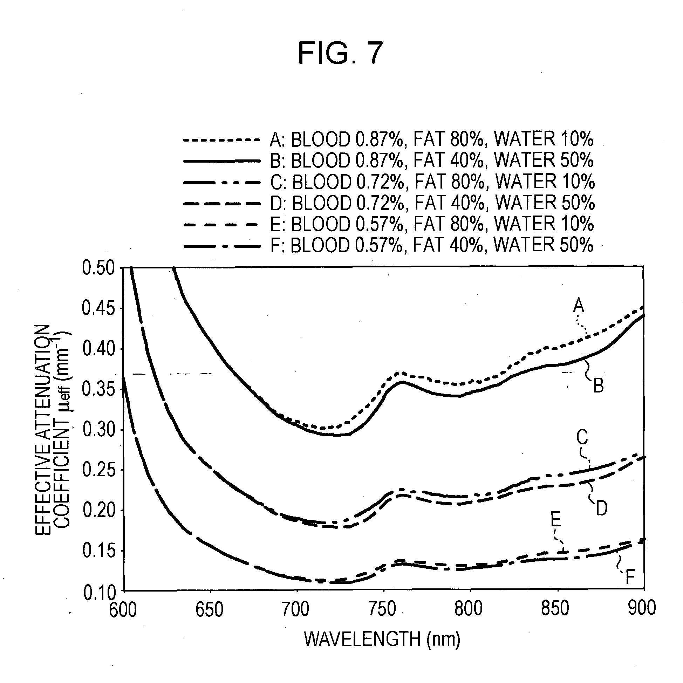

Fig. 7 shows effective attenuation coefficient spectra A to F for different subjects having various component ratios, wherein these effective attenuation coefficient spectra are determined by simulation for various values of component ratios of fat (40 to 80%), water (10 to 50%) and blood (0.57 to 0.87%). Note that in the simulation, the oxygen saturation of the blood is fixed to 75%.

As can be seen from Fig. 7, the shape of the effective attenuation coefficient spectrum varies depending on the component ratio of the subject 300. Therefore, in selection of two wavelengths at which equal effective attenuation coefficients are obtained, the spectrum acquisition apparatus 700 may acquire an effective attenuation coefficient spectrum for each subject. Alternatively, the spectrum acquisition apparatus 700 may select an effective attenuation coefficient spectrum for each subject from data of a plurality of effective attenuation coefficient spectra.

Second Embodiment

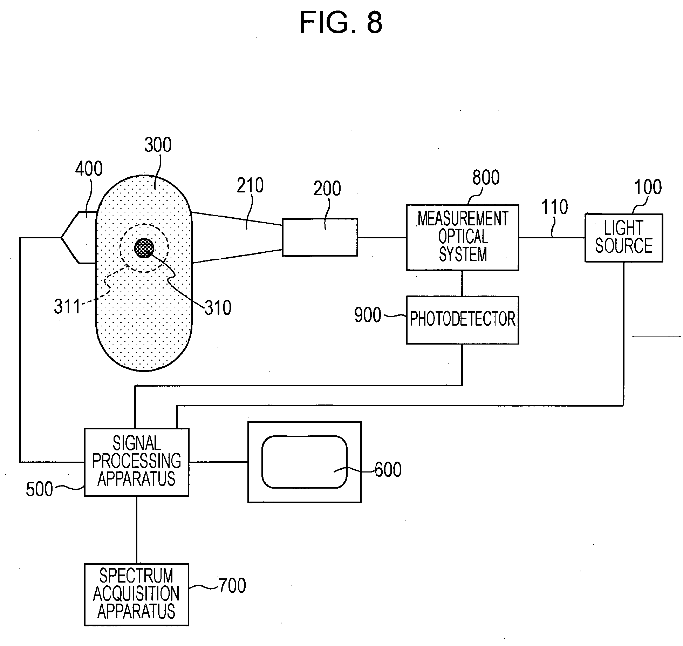

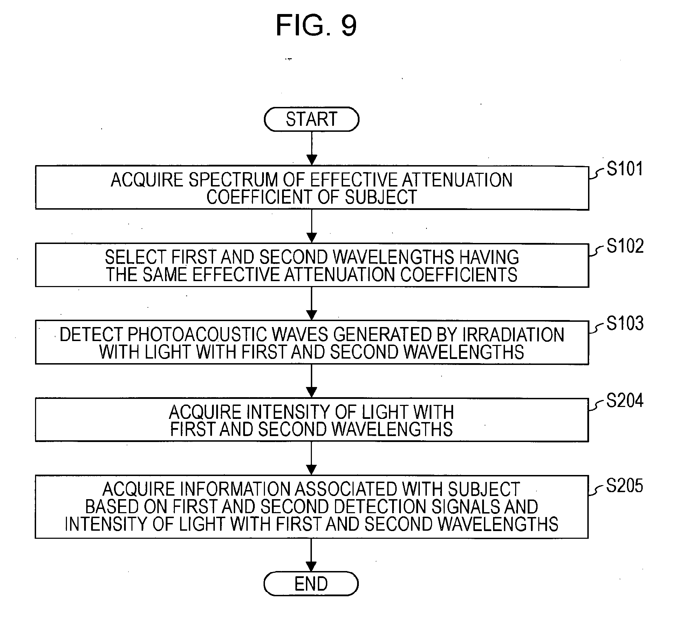

Next, referring to Fig. 8 and Fig. 9, a second embodiment is described below. Fig. 8 is a diagram illustrating a configuration of a subject information acquisition apparatus according to the second embodiment. In this figure, elements similar to those in Fig. 1 are denoted by similar reference numerals and a further description thereof is omitted.

The present embodiment is different from the first embodiment in that a photodetector 900 is additionally provided as a photodetector unit configured to detect light illuminating the subject 300. Furthermore, in the present embodiment, the signal processing apparatus 500 acquires an oxygen saturation of the subject 300 based on the light intensity acquired by the photodetector 900.

Fig. 9 is a flow chart illustrating a subject information acquisition method according to the present embodiment. A further description of processing steps similar to those shown in Fig. 2 is omitted.

S204: Step of acquiring intensity of light with first wavelength and that with second wavelength

In this step, the photodetector 900 detects the light with the first wavelength and the light with the second wavelength and acquires the intensity of the light with the first wavelength and that with the second wavelength.

In the present embodiment, pulsed light 110 is split by a measurement optical system 800 and directed to the photodetector 900.

Note that the measurement optical system 800 may direct illumination light 210, instead of the pulsed light 110, to the photodetector 900.

The measurement optical system 800 may direct all or part of the light to the photodetector 900. In a case where the measurement optical system 800 directs part of the light to the photodetector 900, the signal processing apparatus 500 may acquire the total light intensity of the illumination light 210 based on the light split ratio and the detected light intensity.

In this process of acquiring the total light intensity of the illumination light 210, the signal processing apparatus 500 may take into account an influence of the illumination optical system 200 on the light intensity.

As for the measurement optical system 800, a total reflection mirror, a separate mirror, a bundle fiber, or the like may be employed. The measurement optical system 800 may be realized by a combination of two or more of the above elements. The illumination optical system 200 may be used also as the measurement optical system 800.

The photodetector 900 is an optical power meter, and an optical sensor using a photodiode, a thermal sensor using a thermocouple, a pyroelectric sensor using a pyroelectric material, or the like may be used. In particular, when short pulse light is used, a pyroelectric sensor may be used as the photodetector 900.

S205: Step of acquiring subject information based on the first and second detection signals and intensity of light with first and second wavelengths

In this step, the signal processing apparatus 500 acquires oxygen saturation based on the first detection signal and the second detection signal acquired in step S103 and the intensity of the light with the first wavelength and the intensity of the light with the second wavelength acquired in step S204.

Let $A(\lambda)$ denote the light intensity (surface light intensity) of the surface of the subject, and let $\Phi_r(\lambda, r)$ denote the light intensity distribution in the subject expressed in relative value assuming that the light intensity on the surface of the subject is equal to 1, wherein $\Phi_r(\lambda, r)$ is called a relative light intensity distribution. In the present embodiment, the same illumination optical system 200 is used for all wavelengths, and thus it is assumed that the optical pattern is identical for all wavelengths, that is, the relative light intensity distribution is identical for all wavelengths.

In this situation, according to the present embodiment, the initial pressure is given by Equation (10).

$$\begin{cases} P_0(\lambda_1, r) = \Gamma \cdot \mu_a(\lambda_1, r) \cdot A(\lambda_1) \cdot \Phi_r(r) \\ P_0(\lambda_2, r) = \Gamma \cdot \mu_a(\lambda_2, r) \cdot A(\lambda_2) \cdot \Phi_r(r) \end{cases} \quad (10)$$

By applying the initial pressure in Equation (10) to Equation (4), Equation (11) is obtained which represents the oxygen saturation.

$$StO_2(r) = \frac{-\dfrac{P_0(\lambda_1, r)}{A(\lambda_1)} \varepsilon_{Hb}(\lambda_2) + \dfrac{P_0(\lambda_2, r)}{A(\lambda_2)} \varepsilon_{Hb}(\lambda_1)}{-\dfrac{P_0(\lambda_1, r)}{A(\lambda_1)} \{\varepsilon_{Hb}(\lambda_2) - \varepsilon_{HbO}(\lambda_2)\} + \dfrac{P_0(\lambda_2, r)}{A(\lambda_2)} \{\varepsilon_{Hb}(\lambda_1) - \varepsilon_{HbO}(\lambda_1)\}}$$

$$(11)$$

Thus, according to Equation (11), the signal processing apparatus 500 is capable of determining the oxygen saturation from the light intensity acquired in step S204 and the detection signal acquired in step S103.

Thus, the subject information acquisition method according to the present embodiment allows it to acquire the oxygen saturation without acquiring the light intensity distribution in the subject even in a case where there is a difference in intensity between the two light rays with different wavelengths striking the subject 300. Therefore, in the present embodiment, it is allowed to remove the process of acquiring the light intensity distribution from the process of determining the oxygen saturation.

The signal processing apparatus 500 may be implemented on a computer, and a program including the process described above may be executed by the computer.

Third Embodiment

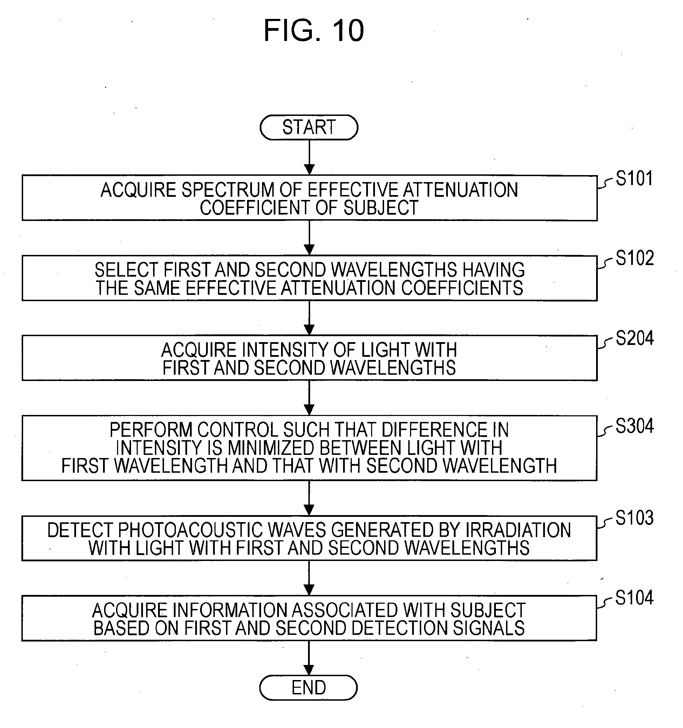

A third embodiment is described below with reference to Fig. 8 and Fig. 10. Fig. 10 is a flow chart illustrating a subject information acquisition method according to the third embodiment. In the following description, processing steps similar to those shown in Fig. 2 or Fig. 9 are omitted.

S304: Step of controlling the intensity of light with the first wavelength and that with the second wavelength.

In this step, the signal processing apparatus 500 serving as the control unit controls the intensity of the light with the first wavelength and the intensity of the light with the second wavelength based on the intensity of light acquired in step S204 so as to minimize the difference between the intensity of the light with first wavelength and the intensity of the light with the second wavelength.

In the present embodiment, the controlling of the light intensity is performed by the signal processing apparatus 500 by controlling the output of the light source 100. Alternatively, the signal processing apparatus 500 may control the illumination optical system 200 to control the light intensity. More specifically, for example, the signal processing apparatus 500 may control an optical filter in the illumination optical system 200 so as to control the intensity of light with the respective wavelengths. Note that the signal processing apparatus 500 may correct the light intensity for either one of light beams or for both light beams.

Instead of the signal processing apparatus 500, another different apparatus may control the output of the light source 100 or the illumination optical system 200.

In this step, the control is performed such that the difference in light intensity between the two wavelengths is minimized, and thus the difference in light intensity distribution in the subject between the two wavelengths is also minimized.

Thus, by controlling the light intensity, it is possible to increase the accuracy of the oxygen saturation acquired in step S103 without acquiring the light intensity distribution.

The signal processing apparatus 500 may be implemented on a computer, and a program including the process described above may be executed by the computer.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

In an apparatus arranged to acquire subject information, a light source (100) is capable of generating light with a first wavelength and alight with a second wavelength different from the first wavelength, an acoustic wave detection unit (400) detects a photoacoustic wave generated by illuminating a subject with light and output a resultant detection signal, and a signal processing unit (500) acquires subject information based on a first detection signal output by the acoustic wave detection unit when the subject is illuminated with the light with the first wavelength and a second detection signal output by the acoustic wave detection unit when the subject is illuminated with the light with the second wavelength. An effective attenuation coefficient of the subject at the first wavelength is equal to an effective attenuation coefficient of the subject at the second wavelength.

**Claims**

1. A subject information acquisition apparatus comprising:

   a light source (100) arranged to be capable of generating light with a first wavelength and light with a second wavelength different from the first wavelength;
   an acoustic wave detection unit (400) arranged to detect a photoacoustic wave generated by illuminating a subject with light and output a resultant detection signal; and

a signal processing unit (500) arranged to acquire subject information based on a first detection signal output by the acoustic wave detection unit when the subject is illuminated with the light with the first wavelength and a second detection signal output by the acoustic wave detection unit when the subject is illuminated with the light with the second wavelength, **characterized in that**

the signal processing unit is arranged to set the first wavelength and the second wavelength based on an effective attenuation coefficient spectrum of the subject,

such that an effective attenuation coefficient of the subject at the first wavelength is substantially equal to an effective attenuation coefficient of the subject at the second wavelength.

2. The subject information acquisition apparatus according to claim 1, further comprising a spectrum acquisition unit (700) arranged to acquire the effective attenuation coefficient spectrum.

3. The subject information acquisition apparatus according to one of claims 1 to 2, further comprising an illumination optical unit (200) arranged to obtain optical patterns equal for both the light with the first wavelength and the light with the second wavelength.

4. The subject information acquisition apparatus according to one of claims 1 to 3, further comprising:

a photodetector unit (900) arranged to acquire intensity of the light with the first wavelength and intensity of the light with the second wavelength; and

a control unit (500) arranged to control the intensity of the light with the first wavelength or the intensity of the light with the second wavelength based on the intensity of the light with first wavelength and the intensity of the light with second wavelength acquired by the photodetector unit so as to minimize the difference between the intensity of the light with first wavelength and the intensity of the light with the second wavelength.

5. The subject information acquisition apparatus according to one of claims 1 to 4, wherein the signal processing unit is arranged to:

acquire a first initial pressure distribution in the subject based on the first detection signal;

acquire a second initial pressure distribution in the subject based on the second detection signal; and

acquire the subject information based on the first initial pressure distribution and the second initial pressure distribution.

6. The subject information acquisition apparatus according to one of claims 1 to 5, wherein the subject information is an oxygen saturation distribution in the subject.

7. The subject information acquisition apparatus according to one of claims 1 to 6, the signal processing unit selects the first wavelength and the second wavelength from the effective attenuation coefficient spectrum of the subject and sets the first wavelength and the second wavelength selected by the signal processing unit.

8. The subject information acquisition apparatus according to one of claims 1 to 6, further comprising a selecting means arranged so that a user is enabled to select the first wavelength and the second wavelength from the effective attenuation coefficient spectrum of the subject and to output information according to the first and second wavelengths selected by the user,

wherein the signal processing unit is arranged to set the first wavelength and the second wavelength by using the information output from the selecting means.

9. The subject information acquisition apparatus according to one of claims 1 to 8, wherein the signal processing unit is arranged to set the first wavelength and the second wavelength which allow it to distinguish between absorbers having oxygen saturations different by 10%.

10. A method of acquiring subject information, comprising:

illuminating a subject with light with a first wavelength,

obtaining a first detection signal by detecting a photoacoustic wave generated by illuminating the subject with light with the first wavelength,

illuminating the subject with light with a second wavelength different from the first wavelength,

obtaining a second detection signal by detecting a photoacoustic wave generated by illuminating the subject with light with the second wavelength,
acquiring subject information based on the first detection signal and the second detection signal, **characterized by**
setting the first wavelength and the second wavelength based on an effective attenuation coefficient spectrum of the subject,
such that an effective attenuation coefficient of the subject at the first wavelength is substantially equal to an effective attenuation coefficient of the subject at the second wavelength.

11. The method according to claim 10, further comprising acquiring the effective attenuation coefficient spectrum of the subject.

12. The method according to one of claims 10 to 11, controlling intensity of the light with the first wavelength or intensity of the light with the second wavelength based on the intensity of the light with first wavelength and the intensity of the light with second wavelength so as to minimize the difference between the intensity of the light with first wavelength and the intensity of the light with the second wavelength.

13. The method according to one of claims 10 to 12, comprising:

   acquiring a first initial pressure distribution in the subject based on the first detection signal detected when the subject is illuminated with the light with the first wavelength;
   acquiring a second initial pressure distribution in the subject based on the second detection signal detected when the subject is illuminated with the light with the second wavelength; and
   acquiring the subject information based on the first initial pressure distribution and the second initial pressure distribution.

14. The method according to one of claims 10 to 13, wherein the subject information is an oxygen saturation distribution in the subject.

15. The method for acquiring subject information according to one of claims 10 to 14, further comprising selecting the first wavelength and the second wavelength from an effective attenuation coefficient spectrum of the subject.

16. The method for acquiring subject information according to one of claims 10 to 15, further comprising inputting information according to the first wavelength or the second wavelength selected from the effective attenuation coefficient spectrum of the subject by an user,
wherein in the setting the first wavelength and the second wavelength, the setting is performed by using the information.

17. The method for acquiring subject information according to one of claims 10 to 16, wherein the first wavelength and the second wavelength allow it to distinguish between absorbers having oxygen saturations different by 10%.

18. A program, which, when executed on the signal processing unit of the apparatus of claim 1, causes the apparatus of claim 1 to execute the steps of
setting a first wavelength and a second wavelength different from the first wavelength based on an effective attenuation coefficient spectrum of the subject, such that an effective attenuation coefficient of a subject at the first wavelength is substantially equal to an effective attenuation coefficient of the subject at the second wavelength, and
acquiring subject information based on a first detection signal obtained based on a photoacoustic wave generated by illuminating the subject with light with the first wavelength set in the step of setting and a second detection signal obtained based on a photoacoustic wave generated by illuminating the subject with light with the second wavelength set in the step of setting.

**Patentansprüche**

1. Gegenstandsinformationserhaltevorrichtung mit:

   einer Lichtquelle (100), die angebracht ist, sodass sie in der Lage ist, Licht mit einer ersten Wellenlänge und Licht mit einer zweiten Wellenlänge, die verschieden von der ersten Wellenlänge ist, zu erzeugen;
   einer Akustischewellenerfassungseinheit (400), die angebracht ist, eine fotoakustische Welle zu erfassen, die durch Beleuchten eines Gegenstands mit Licht erzeugt wird, und ein sich ergebendes Erfassungssignal auszugeben; und

eine Signalverarbeitungseinheit (500), die angebracht ist, eine Gegenstandsinformation basierend auf einem ersten Erfassungssignal das durch die Akustischewellenerfassungseinheit ausgegeben wird, wenn der Gegenstand mit dem Licht mit der ersten Wellenlänge bestrahlt wird, und ein zweites Erfassungssignal, das durch die Akustischewellenerfassungseinheit ausgegeben wird, wenn der Gegenstand mit dem Licht mit der zweiten Wellenlänge beleuchtet wird, zu erhalten,

**dadurch gekennzeichnet, dass**

die Signalverarbeitungseinheit angebracht ist, die erste Wellenlänge und die zweite Wellenlänge basierend auf einem effektiven Abschwächungskoeffizientenspektrum des Gegenstands einzustellen,

sodass ein effektiver Abschwächungskoeffizient des Gegenstands bei der ersten Wellenlänge im Wesentlichen gleich einem effektiven Abschwächungskoeffizienten des Gegenstands bei der zweiten Wellenlänge ist.

2. Gegenstandsinformationserhaltevorrichtung nach Anspruch 1, ferner mit einer Spektrumserhalteeinheit (7), die angebracht ist, das effektive Abschwächungskoeffizientenspektrum zu erhalten.

3. Gegenstandsinformationserhaltevorrichtung nach einem der Ansprüche 1 bis 2, ferner mit einer optischen Beleuchtungseinheit (200), die angebracht ist, optische Muster zu erhalten, die sowohl für das Licht mit der ersten Wellenlänge als auch das Licht mit der zweiten Wellenlänge gleich sind.

4. Gegenstandsinformationserhaltevorrichtung nach einem der Ansprüche 1 bis 3, ferner mit:

einer Fotodetektoreinheit (900), die angebracht ist, eine Intensität des Lichts mit der ersten Wellenlänge und eine Intensität des Lichts mit der zweiten Wellenlänge zu erhalten; und

einer Steuerungseinheit (500), die angebracht ist, die Intensität des Lichts mit der ersten Wellenlänge oder die Intensität des Lichts mit der zweiten Wellenlänge basierend auf der Intensität des Lichts mit der ersten Wellenlänge und der Intensität des Lichts mit der zweiten Wellenlänge, die durch den Fotodetektor erhalten werden, zu steuern, um so die Differenz zwischen der Intensität des Lichts mit der ersten Wellenlänge und der Intensität des Lichts mit der zweiten Wellenlänge zu minimieren.

5. Gegenstandsinformationserhaltevorrichtung nach einem der Ansprüche 1 bis 4, wobei die Signalverarbeitungseinheit angebracht ist um:

eine erste initiale Druckverteilung in dem Gegenstand basierend auf dem ersten Erfassungssignal zu erhalten;

eine zweite initiale Druckverteilung in dem Gegenstand basierend auf dem zweiten Erfassungssignal zu erhalten; und

die Gegenstandsinformation basierend auf der ersten initialen Druckverteilung und der zweiten initialen Druckverteilung zu erhalten.

6. Gegenstandsinformationserhaltevorrichtung nach einem der Ansprüche 1 bis 5, wobei die Gegenstandsinformation eine Sauerstoffsättigungsverteilung in dem Gegenstand ist.

7. Gegenstandsinformationserhaltevorrichtung nach einem der Ansprüche 1 bis 6, wobei die Signalverarbeitungseinheit die erste Wellenlänge und die zweite Wellenlänge aus dem effektiven Abschwächungskoeffizientenspektrum des Gegenstands auswählt und die erste Wellenlänge und die zweite Wellenlänge einstellt, die von der Signalverarbeitungseinheit ausgewählt werden.

8. Gegenstandsinformationserhaltevorrichtung nach einem der Ansprüche 1 bis 6, ferner mit einer Auswahleinrichtung, die angebracht ist, sodass ein Nutzer in die Lage versetzt wird, die erste Wellenlänge und die zweite Wellenlänge aus dem effektiven Abschwächungskoeffizientenspektrum des Gegenstands auszuwählen, und dass eine Information gemäß den ersten und zweiten Wellenlängen, die von dem Nutzer ausgewählt werden, ausgegeben wird, wobei die Signalverarbeitungseinheit angebracht ist, die erste Wellenlänge und die zweite Wellenlänge unter Verwendung der von der Auswahleinrichtung ausgegebenen Information einzustellen.

9. Gegenstandsinformationserhaltevorrichtung nach einem der Ansprüche 1 bis 8, wobei die Signalverarbeitungseinheit angebracht ist, die erste Wellenlänge und die zweite Wellenlänge einzustellen, die es ermöglichen, zwischen Absorbern mit Sauerstoffsättigungen, die um 10% verschieden sind, zu unterscheiden.

**10.** Verfahren zum Erhalten einer Gegenstandsinformation mit:

Beleuchten eines Gegenstands mit Licht mit einer ersten Wellenlänge,
Erhalten eines ersten Erfassungssignals durch Erfassen einer fotoakustischen Welle, die durch Beleuchten des Objekts mit Licht mit der ersten Wellenlänge erzeugt wird,
Beleuchten des Objekts mit Licht mit einer zweiten Wellenlänge, die verschieden von der ersten Wellenlänge ist,
Erhalten eines zweiten Erfassungssignals durch Erfassen einer fotoakustischen Welle, die durch Beleuchten des Objekts mit Licht mit der zweiten Wellenlänge erzeugt wird,
Erhalten einer Gegenstandsinformation basierend auf dem ersten Erfassungssignal und dem zweiten Erfassungssignal,
**gekennzeichnet durch**
Einstellen der ersten Wellenlänge und der zweiten Wellenlänge basierend auf einem effektiven Abschwächungskoeffizientenspektrum des Gegenstands,
sodass ein effektiver Abschwächungskoeffizient des Gegenstands bei der ersten Wellenlänge im Wesentlichen gleich einem effektiven Abschwächungskoeffizienten des Gegenstands bei der zweiten Wellenlänge ist.

**11.** Verfahren nach Anspruch 10, ferner mit Erhalten des effektiven Abschwächungskoeffizientenspektrums des Gegenstands.

**12.** Verfahren nach einem der Ansprüche 10 bis 11, mit Steuern einer Intensität des Lichts mit der ersten Wellenlänge oder einer Intensität des Lichts mit der zweiten Wellelänge basierend auf der Intensität des Lichts mit der ersten Wellenlänge und der Intensität des Lichts mit der zweiten Wellenlänge, um so die Differenz zwischen der Intensität des Lichts mit der ersten Wellenlänge und der Intensität des Lichts mit der zweiten Wellenlänge zu minimieren.

**13.** Verfahren nach einem der Ansprüche 10 bis 12, mit:

Erhalten einer ersten initialen Druckverteilung in dem Gegenstand basierend auf dem ersten Erfassungssignal, das erhalten wird, wenn der Gegenstand mit dem Licht mit der ersten Wellenlänge beleuchtet wird;
Erhalten einer zweiten initialen Druckverteilung in dem Gegenstand basierend auf dem zweiten Erfassungssignal, das erfasst wird, wenn der Gegenstand mit dem Licht mit der zweiten Wellenlänge beleuchtet wird; und
Erhalten der Gegenstandsinformation basierend auf der ersten initialen Druckverteilung und der zweiten initialen Druckverteilung.

**14.** Verfahren nach einem der Ansprüche 10 bis 13, wobei die Gegenstandsinformation eine Sauerstoffsättigungsverteilung in dem Gegenstand ist.

**15.** Verfahren zum Erhalten einer Gegenstandsinformation nach einem der Ansprüche 10 bis 14, ferner mit Auswählen der ersten Wellenlänge und der zweiten Wellenlänge aus einem effektiven Abschwächungskoeffizientenspektrum des Gegenstands.

**16.** Verfahren zum Erhalten einer Gegenstandsinformation nach einem der Ansprüche 10 bis 15, ferner mit Eingeben einer Information gemäß der ersten Wellenlänge oder der zweiten Wellenlänge, die aus dem effektiven Abschwächungskoeffizientenspektrum des Gegenstands durch einen Nutzer ausgewählt werden, wobei bei dem Einstellen der ersten Wellenlänge und der zweiten Wellenlänge das Einstellen unter Verwendung der Information durchgeführt wird.

**17.** Verfahren zum Erhalten einer Gegenstandsinformation nach einem der Ansprüche 10 bis 16, wobei die ersten Wellenlänge und die zweite Wellenlänge es ermöglichen, zwischen Absorbern mit Sauerstoffsättigungen zu unterscheiden, die um 10% verschieden sind.

**18.** Ein Programm, das, wenn es auf der Signalverarbeitungseinheit der Vorrichtung nach Anspruch 1 ausgeführt wird, verursacht, dass die Vorrichtung des Anspruchs 1 die Schritte durchführt:

Einstellen einer ersten Wellenlänge und Einstellen einer zweiten Wellenlänge, die verschieden von der ersten Wellenlänge ist, basierend auf einem effektiven Abschwächungskoeffizientenspektrum des Gegenstands, sodass ein effektiver Abschwächungskoeffizient eines Gegenstands bei der ersten Wellenlänge im Wesentlichen gleich einem effektiven Abschwächungskoeffizienten des Gegenstands bei der zweiten Wellenlänge ist, und Erhalten einer Gegenstandsinformation basierend auf einem ersten Erfassungssignal, das basierend auf einer fotoakustischen Welle erhalten wird, die durch Beleuchten des Gegenstands mit Licht mit der ersten Wellenlänge erzeugt wird, die in dem Schritt des Einstellens eingestellt wird, und auf einem zweiten Erfassungssignal, das basierend auf einer fotoakustischen Welle, die durch Beleuchten des Gegenstands mit Licht mit der zweiten Wellenlänge, die in dem Schritt des Einstellens eingestellt wird, erzeugt wird.

## Revendications

**1.** Appareil d'acquisition d'informations de sujet comprenant :

une source de lumière (100) agencée pour être capable de générer une lumière avec une première longueur d'onde et une lumière avec une deuxième longueur d'onde différente de la première longueur d'onde ;
une unité de détection d'onde acoustique (400) agencée pour détecter une onde photoacoustique générée en éclairant un sujet par la lumière et pour délivrer un signal de détection résultant ; et
une unité de traitement de signal (500) agencée pour acquérir des informations de sujet sur la base d'un premier signal de détection délivré par l'unité de détection d'onde acoustique lorsque le sujet est éclairé par la lumière avec la première longueur d'onde et d'un deuxième signal de détection délivré par l'unité de détection d'onde acoustique lorsque le sujet est éclairé par la lumière avec la deuxième longueur d'onde, **caractérisé en ce que** l'unité de traitement de signal est agencée pour établir la première longueur d'onde et la deuxième longueur d'onde sur la base d'un spectre de coefficient d'atténuation efficace du sujet, de sorte qu'un coefficient d'atténuation efficace du sujet à la première longueur d'onde soit sensiblement égal à un coefficient d'atténuation efficace du sujet à la deuxième longueur d'onde.

**2.** Appareil d'acquisition d'informations de sujet selon la revendication 1, comprenant en outre une unité d'acquisition de spectre (700) agencée pour acquérir le spectre de coefficient d'atténuation efficace.

**3.** Appareil d'acquisition d'informations de sujet selon l'une des revendications 1 et 2, comprenant en outre une unité optique d'éclairage (200) agencée pour obtenir des motifs optiques identiques à la fois pour la lumière avec la première longueur d'onde et la lumière avec la deuxième longueur d'onde.

**4.** Appareil d'acquisition d'informations de sujet selon l'une des revendications 1 à 3, comprenant en outre :

une unité de photodétecteur (900) agencée pour acquérir l'intensité de la lumière avec la première longueur d'onde et l'intensité de la lumière avec la deuxième longueur d'onde ; et
une unité de commande (500) agencée pour commander l'intensité de la lumière avec la première longueur d'onde ou l'intensité de la lumière avec la deuxième longueur d'onde sur la base de l'intensité de la lumière avec la première longueur d'onde et de l'intensité de la lumière avec la deuxième longueur d'onde acquises par l'unité de photodétecteur de manière à réduire à un minimum la différence entre l'intensité de la lumière avec la première longueur d'onde et l'intensité de la lumière avec la deuxième longueur d'onde.

**5.** Appareil d'acquisition d'informations de sujet selon l'une des revendications 1 à 4, dans lequel l'unité de traitement de signal est agencée pour :

acquérir une première répartition de pression initiale dans le sujet sur la base du premier signal de détection ;
acquérir une deuxième répartition de pression initiale dans le sujet sur la base du deuxième signal de détection ; et
acquérir les informations de sujet sur la base de la première répartition de pression initiale et de la deuxième répartition de pression initiale.

**6.** Appareil d'acquisition d'informations de sujet selon l'une des revendications 1 à 5, dans lequel les informations de sujet sont une répartition de la saturation en oxygène dans le sujet.

**7.** Appareil d'acquisition d'informations de sujet selon l'une des revendications 1 à 6, dans lequel l'unité de traitement de signal sélectionne la première longueur d'onde et la deuxième longueur d'onde à partir du spectre de coefficient d'atténuation efficace du sujet et établit la première longueur d'onde et la deuxième longueur d'onde sélectionnées par l'unité de traitement de signal.

**8.** Appareil d'acquisition d'informations de sujet selon l'une des revendications 1 à 6, comprenant en outre des moyens de sélection agencés de sorte qu'un utilisateur soit capable de sélectionner la première longueur d'onde et la deuxième longueur d'onde à partir du spectre de coefficient d'atténuation efficace du sujet et pour délivrer des informations conformément aux première et deuxième longueurs d'onde sélectionnées par l'utilisateur, dans lequel l'unité de traitement de signal est agencée pour établir la première longueur d'onde et la deuxième longueur d'onde en utilisant les informations délivrées par les moyens de sélection.

9. Appareil d'acquisition d'informations de sujet selon l'une des revendications 1 à 8, dans lequel l'unité de traitement de signal est agencée pour établir la première longueur d'onde et la deuxième longueur d'onde qui lui permettent d'effectuer une distinction entre des absorbeurs ayant des saturations en oxygène qui diffèrent de 10 %.

10. Procédé d'acquisition d'informations de sujet, comprenant :

l'éclairage d'un sujet par une lumière avec une première longueur d'onde,
l'obtention d'un premier signal de détection en détectant une onde photoacoustique générée en éclairant le sujet par la lumière avec la première longueur d'onde,
l'éclairage du sujet par une lumière avec une deuxième longueur d'onde différente de la première longueur d'onde,
l'obtention d'un deuxième signal de détection en détectant une onde photoacoustique générée en éclairant le sujet par la lumière avec la deuxième longueur d'onde,
l'acquisition d'informations de sujet sur la base du premier signal de détection et du deuxième signal de détection, **caractérisé par**
l'établissement de la première longueur d'onde et de la deuxième longueur d'onde sur la base d'un spectre de coefficient d'atténuation efficace du sujet,
de sorte qu'un coefficient d'atténuation efficace du sujet à la première longueur d'onde soit sensiblement égal à un coefficient d'atténuation efficace du sujet à la deuxième longueur d'onde.

11. Procédé selon la revendication 10, comprenant en outre l'acquisition du spectre de coefficient d'atténuation efficace du sujet.

12. Procédé selon l'une des revendications 10 et 11, commandant l'intensité de la lumière avec la première longueur d'onde ou l'intensité de la lumière avec la deuxième longueur d'onde sur la base de l'intensité de la lumière avec la première longueur d'onde et de l'intensité de la lumière avec la deuxième longueur d'onde de manière à réduire à un minimum la différence entre l'intensité de la lumière avec la première longueur d'onde et l'intensité de la lumière avec la deuxième longueur d'onde.

13. Procédé selon l'une des revendications 10 à 12, comprenant :

l'acquisition d'une première répartition de pression initiale dans le sujet sur la base du premier signal de détection détecté lorsque le sujet est éclairé par la lumière avec la première longueur d'onde ;
l'acquisition d'une deuxième répartition de pression initiale dans le sujet sur la base du deuxième signal de détection détecté lorsque le sujet est éclairé par la lumière avec la deuxième longueur d'onde ; et
l'acquisition des informations de sujet sur la base de la première répartition de pression initiale et de la deuxième répartition de pression initiale.

14. Procédé selon l'une des revendications 10 à 13, dans lequel les informations de sujet sont une répartition de la saturation en oxygène dans le sujet.

15. Procédé pour acquérir des informations de sujet selon l'une des revendications 10 à 14, comprenant en outre la sélection de la première longueur d'onde et de la deuxième longueur d'onde à partir d'un spectre de coefficient d'atténuation efficace du sujet.

16. Procédé pour acquérir des informations de sujet selon l'une des revendications 10 à 15, comprenant en outre l'entrée d'informations en fonction de la première longueur d'onde ou de la deuxième longueur d'onde sélectionnée à partir du spectre de coefficient d'atténuation efficace du sujet par un utilisateur, dans lequel, lors de l'établissement de la première longueur d'onde et de la deuxième longueur d'onde, l'établissement est effectué en utilisant les informations.

17. Procédé pour acquérir des informations de sujet selon l'une des revendications 10 à 16, dans lequel la première longueur d'onde et la deuxième longueur d'onde lui permettent d'effectuer une distinction entre des absorbeurs ayant des saturations en oxygène qui diffèrent de 10 %.

18. Programme, qui, lorsqu'il est exécuté sur l'unité de traitement de signal de l'appareil selon la revendication 1, amène l'appareil selon la revendication 1 à exécuter les étapes

    d'établissement d'une première longueur d'onde et d'une deuxième longueur d'onde différente de la première longueur d'onde sur la base d'un spectre de coefficient d'atténuation efficace du sujet, de sorte qu'un coefficient d'atténuation efficace d'un sujet à la première longueur d'onde soit sensiblement égal à un coefficient d'atténuation efficace du sujet à la deuxième longueur d'onde, et

    d'acquisition des informations de sujet sur la base d'un premier signal de détection obtenu sur la base d'une onde photoacoustique générée en éclairant le sujet par la lumière avec la première longueur d'onde établie à l'étape d'établissement et d'un deuxième signal de détection obtenu sur la base d'une onde photoacoustique générée en éclairant le sujet par la lumière avec la deuxième longueur d'onde établie à l'étape d'établissement.

# FIG. 1

# FIG. 2

```
        ( START )
            │
            ▼
┌───────────────────────────────────────────┐
│  ACQUIRE SPECTRUM OF EFFECTIVE ATTENUATION │── S101
│          COEFFICIENT OF SUBJECT            │
└───────────────────────────────────────────┘
            │
            ▼
┌───────────────────────────────────────────┐
│  SELECT FIRST AND SECOND WAVELENGTHS HAVING│── S102
│  THE SAME EFFECTIVE ATTENUATION COEFFICIENTS│
└───────────────────────────────────────────┘
            │
            ▼
┌───────────────────────────────────────────┐
│ DETECT PHOTOACOUSTIC WAVES GENERATED BY    │── S103
│ IRRADIATION WITH LIGHT WITH FIRST AND      │
│ SECOND WAVELENGTHS                         │
└───────────────────────────────────────────┘
            │
            ▼
┌───────────────────────────────────────────┐
│  ACQUIRE INFORMATION ASSOCIATED WITH SUBJECT│── S104
│  BASED ON FIRST AND SECOND DETECTION SIGNALS│
└───────────────────────────────────────────┘
            │
            ▼
         ( END )
```

# FIG. 3

Graph: EFFECTIVE ATTENUATION COEFFICIENT $\mu_{eff}$ (mm$^{-1}$) versus WAVELENGTH (nm)

# FIG. 4

EP 2 554 115 B1

## FIG. 5

## FIG. 6

## FIG. 7

------- A: BLOOD 0.87%, FAT 80%, WATER 10%
——— B: BLOOD 0.87%, FAT 40%, WATER 50%
—··— C: BLOOD 0.72%, FAT 80%, WATER 10%
———— D: BLOOD 0.72%, FAT 40%, WATER 50%
— — — — E: BLOOD 0.57%, FAT 80%, WATER 10%
—·—— F: BLOOD 0.57%, FAT 40%, WATER 50%

# FIG. 8

# FIG. 9

```
                    ( START )
                        │
                        ▼
┌────────────────────────────────────────────────┐  ┌S101
│    ACQUIRE SPECTRUM OF EFFECTIVE ATTENUATION     │
│              COEFFICIENT OF SUBJECT              │
└────────────────────────────────────────────────┘
                        │
                        ▼
┌────────────────────────────────────────────────┐  ┌S102
│     SELECT FIRST AND SECOND WAVELENGTHS HAVING   │
│     THE SAME EFFECTIVE ATTENUATION COEFFICIENTS  │
└────────────────────────────────────────────────┘
                        │
                        ▼
┌────────────────────────────────────────────────┐  ┌S103
│ DETECT PHOTOACOUSTIC WAVES GENERATED BY IRRADIATION │
│   WITH LIGHT WITH FIRST AND SECOND WAVELENGTHS   │
└────────────────────────────────────────────────┘
                        │
                        ▼
┌────────────────────────────────────────────────┐  ┌S204
│          ACQUIRE INTENSITY OF LIGHT WITH         │
│           FIRST AND SECOND WAVELENGTHS           │
└────────────────────────────────────────────────┘
                        │
                        ▼
┌────────────────────────────────────────────────┐  ┌S205
│     ACQUIRE INFORMATION ASSOCIATED WITH SUBJECT  │
│     BASED ON FIRST AND SECOND DETECTION SIGNALS AND │
│ INTENSITY OF LIGHT WITH FIRST AND SECOND WAVELENGTHS │
└────────────────────────────────────────────────┘
                        │
                        ▼
                     ( END )
```

# FIG. 10

START

ACQUIRE SPECTRUM OF EFFECTIVE ATTENUATION
COEFFICIENT OF SUBJECT ⎫ S101

SELECT FIRST AND SECOND WAVELENGTHS HAVING
THE SAME EFFECTIVE ATTENUATION COEFFICIENTS ⎫ S102

ACQUIRE INTENSITY OF LIGHT WITH
FIRST AND SECOND WAVELENGTHS ⎫ S204

PERFORM CONTROL SUCH THAT DIFFERENCE IN
INTENSITY IS MINIMIZED BETWEEN LIGHT WITH
FIRST WAVELENGTH AND THAT WITH SECOND WAVELENGTH ⎫ S304

DETECT PHOTOACOUSTIC WAVES GENERATED BY IRRADIATION
WITH LIGHT WITH FIRST AND SECOND WAVELENGTHS ⎫ S103

ACQUIRE INFORMATION ASSOCIATED WITH SUBJECT
BASED ON FIRST AND SECOND DETECTION SIGNALS ⎫ S104

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010088627 A
- US 20070232911 A1
- US 7515948 B1

### Non-patent literature cited in the description

- **X. WANG ; L. V. WANG et al.** Functional Photoacoustic tomography for non-invasive imaging of cerebral blood oxygenation and blood volume in rat brain in vivo. *Proc. of SPIE,* 2005, vol. 5697
- **MIN QU.** *Proceedings of the SPIE - The International Society for Optical Engineering SPIE,* 2010, vol. 7564
- **NATASHA SHAH ; JESSICA GIBBS ; DULCY WOLVERTON ; ALBERT CERUSSI ; NOLA HYLTON ; BRUCE J TROMBERG.** Combined diffuse optical spectroscopy and contrast-enhanced magnetic resonance imaging for monitoring breast cancer neoadjuvant chemotherapy: a case study. *Journal of Biomedical Optics,* 2005, vol. 10 (5), 051503